# EUROPÄISCHE PATENTSCHRIFT

(11) **EP 2 763 705 B1**
(45) Veröffentlichungstag und Bekanntmachung des Hinweises auf die Patenterteilung: **07.11.2018**
(21) Anmeldenummer: 12787348.7
(22) Anmeldetag: 25.09.2012
(51) Int. Cl.: A61L 2/07, A61L 2/24

(54) **VORRICHTUNG ZUM HYGIENISIEREN UND TROCKNEN**
METHOD FOR SANITIZING AND DRYING
DISPOSITIF D'HYGIÉNISATION ET DE SÉCHAGE

(30) Priorität: 05.10.2011 DE 102011114899
(43) Veröffentlichungstag der Anmeldung: 13.08.2014
(73) Patentinhaber: Heckmann, Michael Markus, 69126 Heidelberg (DE); Krueger, Alexander Peter, 69118 Heidelberg (DE)
(72) Erfinder: Heckmann, Michael Markus, 69126 Heidelberg (DE); Krueger, Alexander Peter, 69118 Heidelberg (DE)
(74) Vertreter: Patent- und Rechtsanwälte Ullrich & Naumann
(86) Internationale Anmeldenummer: PCT/DE2012/000943
(87) Internationale Veröffentlichungsnummer: WO 2013/050011

(56) Entgegenhaltungen:
- EP-A1- 1 980 273
- WO-A1-2009/058946
- DE-A1- 2 916 058
- US-A- 2 340 206

## Beschreibung

Die Erfindung betrifft eine Vorrichtung zum Hygienisieren und Trocknen mit einer Kammer, die das zu behandelnde Gut aufnimmt und mit der Aussenluft kommuniziert, mit einer im Bodenbereich der Kammer befindlichen, mit Flüssigkeit befüllbaren und zum Verdampfen der Flüssigkeit elektrisch beheizbaren Wanne, und mit einem elektrisch betriebenen Gebläse zur Erzeugung eines Trocknungsluftstroms durch die Kammer. Eine solche Vorrichtung ist aus der US 2004/0 258 559 A1 bekannt. Sie hat ein Gehäuse, in dem die Wanne und das Gebläse nebeneinander angeordnet sind. Der Trocknungsluftstrom des Gebläses wird durch einen Luftschacht seitlich an der Wanne vorbeigeleitet. Nachteilig dabei ist, dass keine ganz gleichmäßige Dampfbehandlungs- und Trocknungswirkung in der Kammer erzielt wird.

Eine ähnliche Vorrichtung, wenn auch ohne Gebläse, ist aus der US 2004/0 126 274 A1 bekannt. Die US 3 361 517 A ist für den Einsatz feder- und schwerkraftbetätigter Rückschlagventile in einer Vorrichtung zur Dampf-Desinfektion einschlägig.

Eine Vorrichtung der eingangs genannten Art ist auch aus der US 2 340 206 A bekannt. Die Vorrichtung dient zum Sterilisieren und Trocknen von medizinischen Handschuhen, die in einer Kammer auf ein Gestell aufgesteckt werden. Zur Dampferzeugung wird Wasser in einer elektrisch beheizten Wanne verdampft. Zur Erzeugung eines Trocknungsluftstroms dient ein elektrisches Gebläse. Dampf und Trocknungsluftstrom werden durch das Gestell in die darauf steckenden Handschuhe eingeleitet und die Handschuhe zugleich von außen mit dem Dampf bzw. Trocknungsluftstrom beaufschlagt. Das Gebläse ist seitlich neben der Wanne angeordnet. Der Trocknungsluftstrom gelangt von der Seite in den Dampfraum oberhalb der Wanne.

Die DE 29 16 058 A1 betrifft ein Verfahren und eine Vorrichtung zum Trocknen von Sterilisationsgut im Anschluß an eine Dampfsterilisation. Die mit Feuchtigkeit befrachtete Luft wird zirkuliert und außerhalb der Sterilisationskammer gekühlt und getrocknet.

Die EP 1 980 273 A1 bezieht sich auf eine Entkeimungsvorrichtung zum Desinfizieren von Prothesenlinern. In einer elektrisch beheizten Dampferzeugungseinheit wird Wasser verdampft und der Dampf mit einer Dampfführungseinheit in das Innere des Prothesenliners eingeleitet. Zum Trocknen kann mit einem geräteeigenen Gebläse Heißluft bereitgestellt und in den Prothesenliner eingeleitet werden.

Die WO 2009/058 946 A1 betrifft eine Spülmaschine zum Waschen und Trocknen medizinischen Geräts, das nach dem Spülen mit flüssigem Wasser an Ort und Stelle einem warmen Trocknungsluftstrom ausgesetzt wird. Dafür hat die Spülmaschine eine Heizung und ein Gebläse.

Aufgabe der Erfindung ist es, eine Vorrichtung der eingangs genannten Art in Aufbau und Handhabung besonders einfach und bequem zu gestalten und eine gleichmäßige Dampfbehandlungs- und Trocknungswirkung in der Kammer sicherzustellen.

Bei der diese Aufgabe lösenden Vorrichtung ist das Gebläse unter der Wanne angeordnet. Die Wanne ist mit einem zentralen Luftschacht versehen, über den der Trocknungsluftstrom durch die Wanne hindurch in die Kammer geleitet ist. Die Wanne ist zylinderbecherförmig. Sie hat einen Boden, eine umlaufende Seitenwand und einen zentralsymmetrischen, zylinderrohrförmigen Luftschacht, dessen Rohrkörper den Boden mittig und axial durchsetzt und über den oberen Rand der Seitenwand nach oben hinausragt.

Vorteilhafterweise ergibt sich so ein zentraler, aufsteigender Trocknungsluftstrom durch die Kammer. Der Überstand des Luftschachts dient der Betriebssicherheit. Es kann keine Flüssigkeit aus der Wanne in den Luftschacht überlaufen, und auch einem Eindringen von Flüssigkeitsspritzern wird entgegengewirkt.

Bei einer bevorzugten Ausführungsform hat der Luftschacht oben eine Luftaustrittsöffnung, die von einem Rückschlagventil beherrscht wird. Dadurch wird ein Eindringen von Flüssigkeit in den Luftschacht verhindert.

Bevorzugt ist eine Bauform, bei der das Rückschlagventil luftstrombetätigt öffnet und feder- oder schwerkraftbetätigt schließt. Die Ventilbetätigung kann aber auch elektromagnetisch erfolgen.

Bei einer bevorzugten Ausführungsform hat das Rückschlagventil ein Ventilglied, das durch den Luftstrom in Drehung versetzt wird und als rotierender Luftverteiler wirkt.

Bei einer bevorzugten Ausführungsform ist das Gebläse der Vorrichtung elektrisch beheizt, um den Trocknungsluftstrom zu erwärmen. Das Gebläse kann in geeigneter Weise elektrisch oder elektronisch beschaltet sein, um die Heizung manuell oder automatisch ein- und auszuschalten und gegebenenfalls in ihrer Heizleistung kontinuierlich oder in Stufen zu regeln oder zu steuern.

Die Heizung des Gebläses sollte so dimensioniert sein, dass der Trocknungsluftstrom eine Temperatur von 85 °C ± 10 °C erreicht. Die hohe Lufttemperatur dient der Hygiene.

Bei einer bevorzugten Ausführungsform ist der Trocknungsluftstrom gefiltert. Dazu kann ein auswechselbares Filter oder Mikrosieb saugseitig oder druckseitig von dem Gebläse angeordnet sein. Das Filtern der Luft dient der Hygiene.

Bei einer bevorzugten Ausführungsform hat die Vorrichtung einen ersten Betriebszustand für die Dampfdesinfektion, bei dem sich Flüssigkeit in der Wanne befindet, die Wanne beheizt wird und das Gebläse außer Betrieb ist, und einen zweiten Betriebszustand für die Trocknung, bei dem die Wanne leer und unbeheizt und das Gebläse in Betrieb ist. Die Vorrichtung schaltet manuell oder automatisch gesteuert zwischen den beiden Betriebszuständen um. Vorzugsweise ist eine Anzeige für den jeweils herrschenden Betriebszustand vorgesehen.

Bei einer bevorzugten Ausführungsform besteht die Vorrichtung aus einem Set lösbar auf- und ineinander stapelbarer Komponenten, die sich in vorgegebener Reihenfolge vereinzeln und zusammensetzen lassen. Eine druckdichte Abdichtung am Stoß der Komponenten ist nicht vorgesehen. Zu den Komponenten gehören:
- ein Sockel, in dem sich alle elektrische Verbraucher der Vorrichtung, insbesondere das Gebläse und alle elektrischen Heizungen und die elektrische und elektronische Beschaltung und Steuerung der Vorrichtung befinden;
- ein Wannenaufsatz, der die Wanne für die zu verdampfende Flüssigkeit und den zentralen Luftschacht für den Trocknungsluftstrom baulich vereint;
- ein Gehäusemantel;
- ein Siebgestell als Halterung für das zu behandelnde Gut; und
- ein Deckel.

Vorzugsweise ist die Vorrichtung insgesamt zylindertopfförmig. Die Komponenten des Set sind im wesentlichen zentralsymmetrisch zu der Zylindertopfhauptachse aufgebaut, so dass sie sich koaxial auf- und ineinander stapeln lassen.

Die Erfindung wird im folgenden anhand von zwei in der Zeichnung dargestellten Ausführungsbeispielen näher erläutert. Es zeigen:
- Fig. 1: in Seitenansicht ein Haushaltsgerät zum kombinierten Desinfizieren und Trocknen von Haushaltsartikeln; das Gerät hat ein Gehäuse mit einem Sockel, einem Wannenaufsatz und einem Siebgestell und einen Deckel;
- Fig. 2: schematisch in entsprechender Seitenansicht, teilweise diametral aufgeschnitten, eine Variante des Haushaltsgeräts mit einem in den Sockel eingelassenen Gebläse; und
- Fig. 3: genauso eine Variante mit einem zusätzlichen, in den Deckel eingelassenen Gebläse.

Das Haushaltsgerät ist zylindertopfförmig. Es steht auf einer Unterlage und hat dazu unten einen kreiszylindrischen Sockel 10, auf den ein hohlzylindrischer Gehäusemantel 12 aufbaut, der oben mit einem Zylinderdeckel 14 verschlossen ist. Der Mantel 12 läßt sich von dem Sockel 10 und der Deckel 14 von dem Mantel 12 abnehmen. Das Innere des Gehäuses bildet den Behandlungsraum für Haushaltsartikel, die durch Beaufschlagung mit heißem Dampf desinfiziert und anschließend getrocknet werden sollen. Es ist hier an Babyflaschen, Sauger, Inhalatoren und deren Teile samt Zubehör zu denken.

Oben auf dem Sockel 10 befindet sich ein zylinderbecherförmiger Wannenaufsatz, der einen ebenen Boden 18 und eine umlaufende, hochgewölbte Seitenwand 20 hat. Der Boden 18 wird von unten elektrisch beheizt. Die Heizung 22 ist darauf ausgelegt, in der Wanne befindliche Flüssigkeit, nämlich Wasser mit oder ohne Zusätze, zu verdampfen.

Der Wannenaufsatz läßt sich zu Reinigungs- und Entkalkungszwecken von dem Sockel 10 lösen.

Der Wannenaufsatz hat einen zentralen, zylinderrohrförmigen Luftschacht. Der Rohrkörper 24 des Luftschachts durchsetzt den Boden 18 der Wanne mittig und axial. Er ragt nach oben über den oberen Rand 26 der Wannenseitenwand 20 hinaus.

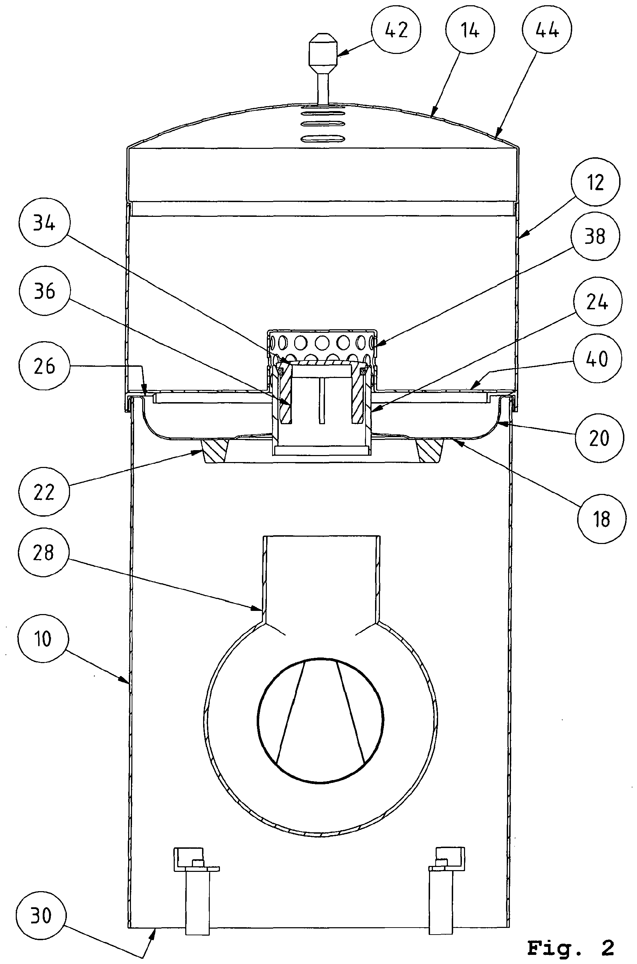

Gemäß Fig. 2 enthält der Sockel 10 ein elektrisch betriebenes Gebläse 28, das Luft durch im Bodenbereich des Sockels 10 befindliche Ansaugöffnungen 30 seitlich von unten in das Gerät einzieht und durch den Luftschacht nach oben befördert. Die Luft kann auf ca. 85 °C erwärmt werden. Sie strömt dazu durch eine elektrische Heizspirale.

Vor der oberen Öffnung des Luftschachts liegt das Ventilglied 34 eines Rückschlagventils, das luftstrombetätigt öffnet und schwerkraftbetätigt schließt. Das Ventilglied 34 ist tellerförmig und mit über den Umfang verteilten, axialen Stegen 36 am Innenmantel des Luftschachts geführt. Das Ventilglied 34 wird durch den Luftstrom in Drehung versetzt, so dass es als rotierender Luftverteiler wirkt.

Auf dem Wannenaufsatz ruht ein Siebgestell 38, das das Innere des Gehäuses einnimmt und die zu behandelnden Haushaltsartikel trägt. Das Siebgestell 38 kann bei geöffnetem Deckel 14 von oben in den Gehäusemantel 12 eingesetzt und daraus entnommen werden. Der Gehäusemantel 12 läßt sich mitsamt dem Siebgestell 38 von dem Sockel 10 abnehmen.

Das Siebgestell 38 hat einen umgekehrt zylinderbecherförmigen Siebboden 40, mit dem es den nach oben vorstehenden Luftschacht überwölbt und dem Ventilglied 34 des Rückschlagventils Platz für seine Öffnungsbewegung nach oben läßt.

Der Deckel 14 des Geräts ist außen auf den Gehäusemantel 12 aufgestülpt und nach oben davon abnehmbar. Er hat außen einen zentralen Griffknopf 42. Der Deckel 14 ist mit Ausblasöffnungen 44 versehen, durch die das Innere des Gehäuses mit der Außenluft kommuniziert. Nach alledem besteht das Gerät aus einer Auf- und Ineinanderschachtelung lösbarer Komponenten, nämlich Sockel 10, Wannenaufsatz, Gehäusemantel 12, Siebgestell 38 und Deckel 14, die in wohldefinierter Reihenfolge vereinzelt und zusammengesetzt werden können. Das gewährleistet eine einfache Bedienung, Wartung und Reinigung. Das Gerät hat kein Drucksystem. Es bedarf daher am Stoß der Komponenten keiner druckfesten Abdichtung.

Bei Inbetriebnahme des Geräts sind alle seine elektrischen Verbraucher, d. h. das Gebläse 28 und die elektrischen Heizungen 22 von Luft und Wasser ausgeschaltet. Deckel 14, Gehäusemantel 12 und Siebgestell 38 werden von dem Sockel 10 abgenommen und die Wanne mit Wasser gefüllt. Es kann destilliertes Wasser oder Leitungswasser gegebenenfalls mit geeigneten Zusätzen verwendet werden. Das Wasser wird einfach von oben in die Wanne gegossen. Dabei kann kein Spritzwasser in den Sockel 10 eindringen, da das Rückschlagventil vor dem Luftschacht geschlossen ist.

Bei versehentlichem Überfüllen der Wanne läuft das Wasser über deren oberen Rand 26 nach außen ab. Selbst bei Versagen des Rückschlagventils kann kein Wasser in den nach oben vorstehenden Luftschacht gelangen.

Nach Aufsetzen des Gehäusemantels 12, Einsetzen des Siebgestells 38 mit den zu desinfizierenden Artikeln und Schließen des Deckels 14 wird das Gerät eingeschaltet. Dabei geht zunächst nur die elektrische Heizung 22 der Wanne in Betrieb. Das Gebläse 28 bleibt ausgeschaltet. Das kann durch entsprechende Betätigung separater Schalter oder eines Stufenschalters, aber auch durch eine Sicherheitsschaltung gewährleistet sein, die ein Einschalten des Gebläses 28 verhindert, solange sich flüssiges Wasser in der Wanne befindet.

Nach restlosem Verdampfen des Wassers wird die Heizung 22 der Wanne ausgeschaltet und zum Trocknen der durch den Dampf desinfizierten Artikel das Gebläse 28 und die Heizung für den Trocknungsluftstrom eingeschaltet. Das geschieht wiederum durch Schalterbetätigung von Hand oder automatisch mit einer geeigneten Steuerfunktion, z. B. zeitabhängig oder in Abhängigkeit von der Temperatur der Wanne. Nach erfolgter Trocknung schaltet das Gerät handbetätigt oder automatisch ab.

Bevorzugt ist eine Zeitsteuerung für alle Schaltfunktionen nach Einschalten von Hand. Nach anfänglichem Dampfdesinfizieren über ein erstes Zeitintervall von beispielsweise 10 min schaltet das Gerät automatisch auf Trocknen über ein zweites Zeitintervall von beispielsweise 15 min um und dann aus. Die Zeitintervalle können nach Beschikkungskapazität, Heiz- und Gebläseleistung des Geräts variieren und herstellerseitig vorgegeben sein.

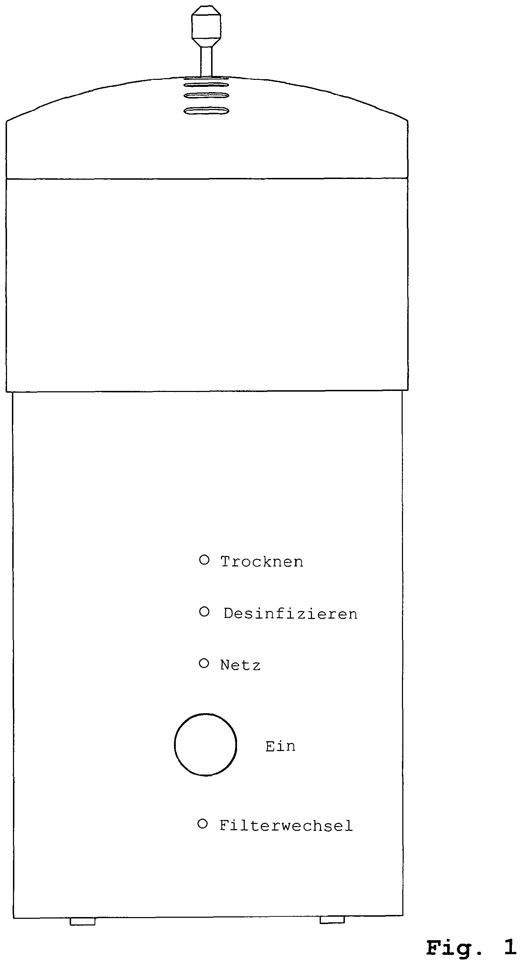

Gemäß Fig. 1 hat das Gerät einen manuell betätigbaren Ein/Aus Schalter und Leuchtanzeigen für die Verbindung mit dem elektrischen Versorgungsnetz, den Betriebszustand "Desinfizieren", den Betriebszustand "Trocknen" und die Empfehlung eines Filterwechsels.

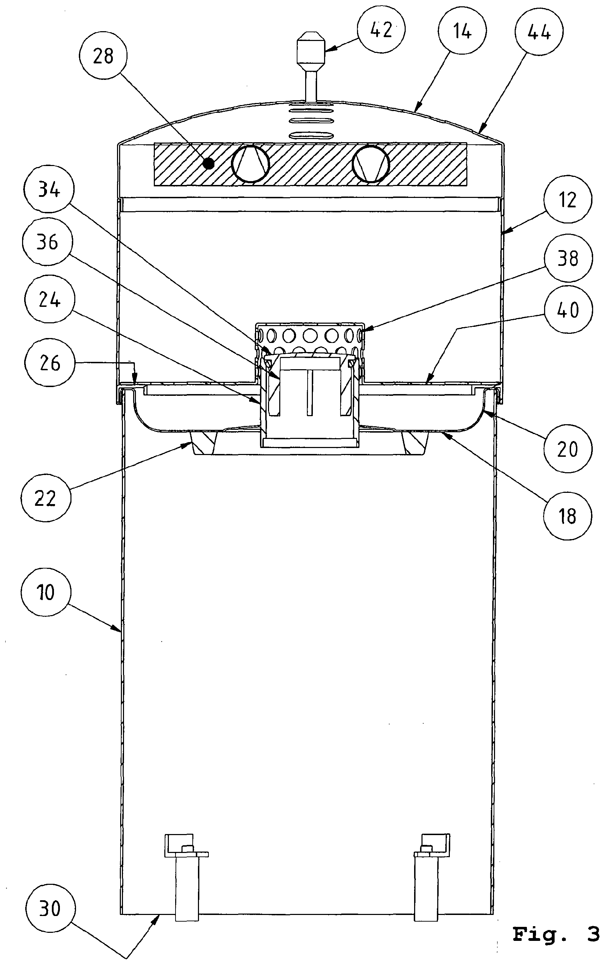

Gemäß Fig. 3 ist zusätzlich ein Gebläse 28 in dem Deckel 14 des Haushaltsgeräts enthalten.

### Liste der Bezugszeichen

- 10: Sockel
- 12: Gehäusemantel
- 14: Deckel
- 18: Boden
- 20: Seitenwand
- 22: Heizung
- 24: Rohrkörper
- 26: Rand
- 28: Gebläse
- 30: Ansaugöffnung
- 34: Ventilglied
- 36: Steg
- 38: Siebgestell
- 40: Siebboden
- 42: Griffknopf
- 44: Ausblasöffnung

## Patentansprüche

1. Vorrichtung zum Hygienisieren und Trocknen mit einer Kammer, die das zu behandelnde Gut aufnimmt und mit der Außenluft kommuniziert, mit einer im Bodenbereich der Kammer befindlichen, mit Flüssigkeit befüllbaren und zum Verdampfen der Flüssigkeit elektrisch beheizbaren Wanne, und mit einem elektrisch betriebenen Gebläse (28) zur Erzeugung eines Trocknungsluftstroms durch die Kammer, **dadurch gekennzeichnet, dass** das Gebläse (28) unter der Wanne angeordnet ist, dass die Wanne mit einem zentralen Luftschacht versehen ist, über den der Trocknungsluftstrom durch die Wanne hindurch in die Kammer geleitet ist, und dass die Wanne zylinderbecherförmig ist und einen Boden (18), eine umlaufende Seitenwand (20) und einen zentralsymmetrischen, zylinderrohrförmigen Luftschacht hat, dessen Rohrkörper (24) den Boden (18) mittig und axial durchsetzt und über den oberen Rand (26) der Seitenwand (20) nach oben hinausragt.

2. Vorrichtung nach Anspruch 1, **dadurch gekennzeichnet, dass** der Luftschacht oben eine Luftaustrittsöffnung hat, die von einem Rückschlagventil beherrscht wird.

3. Vorrichtung nach Anspruch 2, **dadurch gekennzeichnet, dass** das Rückschlagventil luftstrombetätigt öffnet.

4. Vorrichtung nach Anspruch 2 oder 3, **dadurch gekennzeichnet, dass** das Rückschlagventil feder- oder schwerkraftbetätigt schließt.

5. Vorrichtung nach einem der Ansprüche 2 bis 4, **dadurch gekennzeichnet, dass** das Rückschlagventil ein Ventilglied (34) hat, das durch den Luftstrom in Drehung versetzbar ist und als rotierender Luftverteiler wirkt.

6. Vorrichtung nach einem der Ansprüche 1 bis 5, **dadurch gekennzeichnet, dass** der Trocknungsluftstrom elektrisch beheizbar ist.

7. Vorrichtung nach einem der Ansprüche 1 bis 6, **dadurch gekennzeichnet, dass** der Trocknungsluftstrom gefiltert ist.

8. Vorrichtung nach einem der Ansprüche 1 bis 7, **gekennzeichnet durch** einen ersten Betriebszustand für die Dampfdesinfektion, bei dem sich Flüssigkeit in der Wanne befindet, die Wanne beheizt wird und das Gebläse (28) außer Betrieb ist, einen zweiten Betriebszustand für die Trocknung, bei dem die Wanne leer und unbeheizt und das Gebläse (28) in Betrieb ist, und eine Steuerung für die manuelle oder automatische Umschaltung. zwischen den beiden Betriebszuständen.

9. Vorrichtung nach Anspruch 8, **dadurch gekennzeichnet, dass** sie eine Anzeige für den jeweils herrschenden Betriebszustand hat.

10. Vorrichtung nach einem der Ansprüche 1 bis 9, bestehend aus einem Set lösbar auf- und ineinander stapelbarer Komponenten, die in vorgegebener Reihenfolge vereinzelbar und zusammensetzbar sind, dazu gehörend:
- ein Sockel (10), in dem sich alle elektrischen Verbraucher der Vorrichtung, insbesondere das Gebläse (28) und alle elektrischen Heizungen (22) und die elektrische und elektronische Beschaltung und Steuerung der Vorrichtung befinden;
- ein Wannenaufsatz, der die Wanne für die zu verdampfende Flüssigkeit und den zentralen Luftschacht für den Trocknungsluftstrom baulich vereint;
- ein Gehäusemantel (12);
- ein Siebgestell (38) als Halterung für das zu behandelnde Gut; und
- ein Deckel (14).

11. Vorrichtung nach Anspruch 10, **dadurch gekennzeichnet, dass** sie zylindertopfförmig und die Komponenten des Set im wesentlichen zentralsymmetrisch zu der Zylindertopfhauptachse aufgebaut und koaxial auf- und ineinander stapelbar sind.

## Claims

1. Device for sanitising and drying, comprising a chamber that accommodates the material to be treated and communicates with the outside air, comprising a trough that is located in the bottom region of the chamber, can be filled with liquid and can be electrically heated in order to evaporate the liquid, and comprising an electrically operated fan (28) for generating a drying air flow through the chamber, **characterised in that** the fan (28) is arranged beneath the trough, **in that** the trough is provided with a central air duct, by means of which the drying airflow is directed through the trough and into the chamber, and **in that** the trough is cylindrical-cup-shaped and has a bottom (18), a peripheral lateral wall (20) and a centrally symmetrical, cylindrical-tube-shaped air duct, the tube body (24) of which centrally and axially penetrates the bottom (18) and protrudes upwards beyond the upper edge (26) of the lateral wall (20).

2. Device according to claim 1, **characterised in that** the air duct has an air outlet opening in the top, which is controlled by a nonreturn valve.

3. Device according to claim 2, **characterised in that** the nonreturn valve opens in a manner actuated by air flow.

4. Device according to either claim 2 or claim 3, **characterised in that** the nonreturn valve closes in a manner actuated by spring force or gravity.

5. Device according to any one of claims 2 to 4, **characterised in that** the nonreturn valve has a valve member (34) that can be rotated by the air flow and acts as a rotating air distributor.

6. Device according to any one of claims 1 to 5, **characterised in that** the drying air flow can be electrically heated.

7. Device according to any one of claims 1 to 6, **characterised in that** the drying air flow is filtered.

8. Device according to any one of claims 1 to 7, **characterised by** a first operating state for steam disinfection, in which liquid is located in the trough, the trough is heated and the fan (28) is at rest, a second operating state for drying, in which the trough is empty and unheated and the fan (28) is in operation, and a controller for manually or automatically switching between the two operating states.

9. Device according to claim 8, **characterised in that** it has an indicator for the operating state prevailing in each case.

10. Device according to any one of claims 1 to 9, consisting of a set of components that can be detachably stacked one on top of the other and one inside the other and can be separated and combined in a predetermined sequence, including:
- a base (10), in which all electrical loads of the device, in particular the fan (28), and all electrical heating systems (22) and the electrical and electronic wiring and controller of the device are located;
- a trough attachment, which structurally combines the trough for the liquid to be evaporated and the central air duct for the drying air flow;
- a housing shell (12);
- a sieve frame (38) as a holder for the material to be treated; and
- a lid (14).

11. Device according to claim 10, **characterised in that** it is cylindrical-pot-shaped and the components of the set are substantially centrally symmetrical to the cylindrical pot main axis and can be stacked coaxially one on top of the other and one inside the other.

## Revendications

1. Dispositif d'hygiénisation et de séchage avec une chambre, qui reçoit l'article à traiter et qui communique avec l'air ambiant, avec une cuve se trouvant au fond de la chambre, pouvant être remplie d'un liquide et pouvant être chauffée électriquement pour l'évaporation du liquide, et avec un ventilateur électrique (28) pour la production d'un flux d'air de séchage à travers la chambre, **caractérisé en ce que** le ventilateur (28) est disposé sous la cuve, **en ce que** la cuve est munie d'un puits d'air central, par l'intermédiaire duquel le flux d'air de séchage est conduit à travers a chambre, et **en ce que** la cuve présente la forme d'un gobelet cylindrique et comprend un fond (18), une paroi latérale circulaire (20) et un puits d'air à symétrie centrale présentant la forme d'un tube cylindrique, dont le corps tubulaire (24) traverse le fond (18) de manière centrale et axiale et dépasse vers le haut du bord supérieur (26) de la paroi latérale (20).

2. Dispositif selon la revendication 1, **caractérisé en ce que** le puits d'air comprend, en haut, une ouverture de sortie d'air, qui est contrôlée par un clapet anti-retour.

3. Dispositif selon la revendication 2, **caractérisé en ce que** le clapet anti-retour est actionné par le flux d'air.

4. Dispositif selon la revendication 2 ou 3, **caractérisé en ce que** le clapet anti-retour est fermé sous l'effet de la force d'un ressort ou de la gravité.

5. Dispositif selon l'une des revendications 2 à 4, **caractérisé en ce que** le clapet anti-retour comprend un organe de soupape (34) qui peut être mis en rotation par le flux d'air et qui agit comme un distributeur d'air rotatif.

6. Dispositif selon l'une des revendications 1 à 5, **caractérisé en ce que** le flux d'air de séchage peut être chauffé électriquement.

7. Dispositif selon l'une des revendications 1 à 6, **caractérisé en ce que** le flux d'air de séchage est filtré.

8. Dispositif selon l'une des revendications 1 à 7, **caractérisé par** un premier état de fonctionnement pour la désinfection à la vapeur, dans lequel un liquide se trouve dans la cuve, la cuve est chauffée et le ventilateur (28) est arrêté, un deuxième état de fonctionnement pour le séchage, dans lequel la cuve est vide et non chauffée et le ventilateur (28) est en service, et un dispositif de commande pour la commutation manuelle ou automatique entre les deux états de fonctionnement.

9. Dispositif selon la revendication 8, **caractérisé en ce qu'**il comprend un affichage pour l'état de fonctionnement courant.

10. Dispositif selon l'une des revendications 1 à 9, constitué d'un ensemble de composants pouvant être empilés et emboîtés de manière réversible, qui peuvent être séparés et assemblés dans un ordre prédéterminé, comprenant :
- un socle (10), dans lequel se trouvent tous les consommateurs électriques du dispositif, plus particulièrement le ventilateur (28) et tous les chauffages électriques (22) et le câblage électrique et électronique et la commande du dispositif ;
- une pièce de cuve qui regroupe la cuve pour le liquide à évaporer et le puits d'air central pour le flux d'air de séchage ;
- une enveloppe de boîtier (12) ;
- un châssis de tamisage (38) en tant que support pour l'article à traiter ;
- un couvercle (14).

11. Dispositif selon la revendication 10, **caractérisé en ce qu'**il présente la forme d'un pot cylindrique et les composants de l'ensemble sont conçus avec globalement une symétrie centrale par rapport à l'axe principal du pot cylindrique et peuvent être empilés et emboîtés entre eux de manière coaxiale.
